# EUROPEAN PATENT SPECIFICATION

(11) **EP 2 276 776 B1**
(45) Date of publication and mention of the grant of the patent: **29.07.2015**
(21) Application number: 09746780.7
(22) Date of filing: 15.05.2009
(51) Int. Cl.: C07K 1/22, C07K 16/12, B01D 15/38

(54) **METHOD AND AFFINITY COLUMN FOR PURIFYING PROTEINS**
VERFAHREN UND AFFINITÄTSSÄULE ZUR AUFREINIGUNG VON PROTEINEN
PROCÉDÉ ET COLONNE D AFFINITÉ POUR LA PURIFICATION DES PROTÉINES

(30) Priority: 16.05.2008 KR 20080045801; 24.04.2009 KR 20090036008
(43) Date of publication of application: 26.01.2011
(73) Proprietor: Samsung Electronics Co., Ltd., Suwon-si, Gyeonggi-do, 443-742 (KR)
(72) Inventor: LEE, Jaeil, Yongin-si Gyeonggi-do 448-533 (KR); LEE, YoungSun, Yongin-si Gyeonggi-do 446-943 (KR)
(74) Representative: Grünecker Patent- und Rechtsanwälte PartG mbB
(86) International application number: PCT/KR2009/002593
(87) International publication number: WO 2009/139601

(56) References cited:
- EP-A1- 1 685 161
- US-A1- 2005 059 053
- US-A1- 2006 030 696
- ZHAO F ET AL: "Purification and Characterization of the Fusion Protein Trypsin-Streptavidin Expressed in Escherichia coli", JOURNAL OF PROTEIN CHEMISTRY, KLUWER ACADEMIC PUBLISHERS-PLENUM PUBLISHERS, NE, vol. 21, no. 6, 1 August 2002 (2002-08-01) , pages 413-418, XP019284245, ISSN: 1573-4943
- ANTONI POLANOWSKI ET AL: "Non-conventional affinity chromatography of serine proteinases and their inhibitors.", ACTA BIOCHIMICA POLONICA, vol. 50, no. 3, 1 January 2003 (2003-01-01), pages 765-73, XP055001018, ISSN: 0001-527X
- LICHTY J J ET AL: "Comparison of affinity tags for protein purification", PROTEIN EXPRESSION AND PURIFICATION, ACADEMIC PRESS, SAN DIEGO, CA, vol. 41, no. 1, 1 May 2005 (2005-05-01), pages 98-105, XP027429987, ISSN: 1046-5928, DOI: 10.1016/J.PEP.2005.01.019 [retrieved on 2005-03-28]
- ZSOLT LENGYEL, ET AL.: 'Affinity Purification of Recombinant Trypsinogen Using Immobilizted Ecotin' PROTEIN EXPRESSION AND PURIFICATION vol. 12, no. 2, 1998, pages 291 - 294, XP004447552
- GABOR PAL, ET AL.: 'Stable monomeric form of an originally dimeric serine proteinase inhibitor, ecotin, was constructed via site directed mutagenesis' FEBS LETTERS vol. 385, 1996, pages 165 - 170, XP000949191

## Description

### [Technical Field]

This application relates to methods and affinity columns for protein purification.

### [Background Art]

Recently, there have been many trials using proteins in the treatment or diagnosis of diseases. In particular, the market for therapeutic antibodies has been growing rapidly, and many biotech companies are committed to developing such protein therapeutics. The field of recombinant antibody engineering initially involved chimeric antibodies or humanized antibodies, and has now progressed to the level of developing de-immunized antibodies, or fully human antibodies using transformed mice and phage display technology.

Often, proteins for therapy or diagnosis of disease are generated by expressing the proteins during culture of cells transformed with vectors that can express such proteins. Alternatively, such proteins can be overexpressed in recombinant plants or animals. For example, the protein can be expressed in a lactating recombinant animal such that the protein can be obtained from the milk of the transformed animal. In such instances, the protein typically needs to be isolated and purified from the cell culture or milk. When proteins are expressed in plants or microorganisms, isolation and purification will involve extracting the protein from inside the storage organs or cells. Isolation and purification of expressed proteins from any of these sources is often not an easy task. It can require removing or separating protein or DNA from host cells, removing viruses that infect humans, and removing lectin from plants or endotoxin from gram negative bacteria.

A widely used method for purifying antibodies from mammalian cell-culture supernatants or crude protein mixtures, such as serum or ascites fluid, is Protein A affinity chromatography. Protein A binds proteins from many mammalian species. In particular, it binds to the Fc region of immunoglobulins through interaction with the heavy chain. Protein A binds with high affinity to the immunoglobulin, IgG.

Zhao, F. et al., 'Purification and Characterization of the Fusion Protein Trypsin-Streptavidin Expessed in Escherichia coli', Journal of Protein Chemistry, Vol. 21, No. 6, pages 413-418 relates to the purification of the fusion protein trypsin-streptavidin expressed in *E. coli* using a benzamidine sepharose 6B affinity column.

WO 2006/024497 discloses a method for purifying antibody and other product protein concomittant with removing aggregates made up from single product protein species is devised.

### □Disclosure□

### □Technical Problem□

Disclosed herein is a method for purifying a target protein from a sample.

### □Technical Solution□

In an embodiment, the method includes contacting a sample containing a tagged target protein to a support having immobilized thereon a protease inhibitor, wherein the protease inhibitor is ecotin, papaya Kunitz-type trypsin inhibitor, Pepstatin A, leupeptin, Gly-Gly-Tyr-Arg, a2-macroglobulin, a2-antiplasmin, antithrombin III human, alantitrypsin, bdellin, pepsinostreptin, chymostatin, phosphoramidon, isoamylphosphonyl-Gly-L-Pro-L-Ala, or dipotassium 2(R)-2-mercaptomethyl-4-methylpentanoyl-beta-(2-naphthyl)-Ala-Ala amide, wherein the tagged target protein is an antibody covalently bound to a peptide tag and the peptide tag has specific binding affinity for the protease inhibitor, wherein the peptide tag is a) derived from a protease, or b) a fragment of the protease that lacks the proteolysis activity of the protease, a mutant of the protease that lacks the proteolysis activity of the wildtype protease, or a fragment of the mutant, and wherein the contact is under conditions such that the protease inhibitor binds to the peptide tag; removing components of the sample that are not bound; and then removing the target protein from the support.

Also disclosed herein is a fusion protein comprising a peptide tag having specific binding activity to a protease inhibitor and a target protein wherein the target protein is an immunoglobulin heavy chain, and wherein the peptide tag is: a) derived from a protease selected from papain, trypsin, pepsin, chymotrypsin, rennin, cathepsin D, thermolysin, elastase, plasmin, thrombin, urokinase, and collagenase, b) a fragment of the protease that lacks the proteolysis activity of the protease, a mutant of the protease that lacks the proteolysis activity of the wildtype protease, or a fragment of the mutant, or c) a mutant, trypsin. Further disclosed is a recombinant cell comprising an isolated polynucleotide encoding the fusion protein of the invention.

### □Advantageous Effects□

The method and column disclosed herein enable the proteins such as antibodies to be efficiently purified.

### □ Description of Drawings □

Fig. 1 is a diagram showing an embodiment of the process for purifying a target protein disclosed herein.
Fig. 2 is a photograph of a protein gel after electrophoresis of purified ecotin.
Fig. 3 is a photograph of a protein gel after electrophoresis of human cationic trypsinogen (PRSS1) purified using an ecotin affinity column.
Fig. 4 is a diagram representing affinity purification of anti-ecotin antibodies using either a Protein A affinity column or an ecotin affinity column, respectively.
Fig. 5 is a photograph of a protein gel after electrophoresis of anti-ecotin antibodies purified by Protein A affinity chromatography or by ecotin affinity chromatography.
Fig. 6 is a reproduction of part of Swiss-Prot entry P07477, for human cationic trypsinogen (PRSS1) showing the polypeptide sequence (SEQ ID NO:13) and structural features of the polypeptide sequence.
Fig. 7 is a photograph of a protein gel after electrophoresis to confirm expression in HEK293 cells of recombinant anti-EGFR antibodies including a heavy chain tagged with a mutant trypsin tag, which is also mutated to insert a cleavage site for Tobacco Etch Virus (TEV) protease.
Fig. 8 is a photograph of a protein gel after electrophoresis of anti-EGFR antibodies tagged with a mutant trypsin tag purified by ecotin affinity chromatography after expression in CHO-DG44 (DHFR-) cells.

### □Best Mode□

### DETAILED DESCRIPTION

Disclosed herein are methods and affinity chromatography media for protein purification. The method takes advantage of the high binding specificity between a protease and an inhibitor of the protease to yield rapid purification of target proteins, e.g., antibodies.

"Protease" as used in this specification refers to an enzyme that conducts proteolysis, i.e., an enzyme that breaks down protein by hydrolysis of the peptide bond that links the amino acids in a polypeptide chain. Well known classes of proteases include, for example, serine proteases, threonine proteases, cysteine proteases, aspartic acid proteases, metalloproteases, and glutamic acid proteases.

The term "peptide tag" as used in this specification includes any peptide, regardless of its origin or length, that has specific binding affinity to a protease inhibitor. For example, the term "peptide tag" can include a protease having specific binding affinity to a protease inhibitor, a fragment thereof having specific binding affinity to a protease inhibitor, or a mutant thereof having specific binding affinity to a protease inhibitor. An example of a peptide tag that is a fragment of a proteaseis a peptide having specific binding affinity to a protease inhibitor, but lacking the proteolysis activity of the full-length protease. One example of a protease fragment can be the shortest peptide from a protease retaining specific binding affinity to a protease inhibitor, and lacking the proteolysis activity. Further, an example of a peptide tag that is a mutant of a proteaseis a mutant protease polypeptide that lacks the normal proteolysis activity of the wild type protease due to, for example, mutation in the proteolytic active site, but which has specific binding affinity to a protease inhibitor. The length of a peptide tag can be at least 6 contiguous amino acids from the protease, providing that the specific binding affinity for the protease inhibitor is retained and the proteolysis activity is absent.

"Protease inhibitor" as used in this specification refers to any substance that inhibits a protease. A protease inhibitor can be a low molecular weight compound, e.g., the irreversible inhibitors 4-(2-Aminoethyl) benzenesulfonyl fluoride hydrochloride and phenylmethanesulphonylfluoride or phenylmethylsulphonyl fluoride, or a high molecular weight compound, such as a protein.

A "sample containing a tagged protein" as used in this specification refers to any sample containing the target protein, whether naturally derived or artificially prepared. The sample can include mixtures of different liquids, a liquid and a solid, or different solids. Examples of a sample containing a target protein can include blood, serum, ascites fluid, milk, a tissue sample, a cell culture, a cell lysate, or the supernatant of a cell culture.

"Support" as used in this specification refers to anything to which a protease inhibitor can be immobilized to obtain an affinity chromatography medium, regardless of its form or the material from which it is made. Examples include agarose, which is often used in affinity chromatography; cellulose; and polyacrylamide.

"Chromatography medium" as used in this specification refers to a stationary phase used for chromatographic purification regardless of its configuration (column or planar), state (liquid or solid), or the material from which it is made.. C ommon examples of chromatography media used for protein purification include an ion exchange resin, an affinity stationary phase, and a gel permeation stationary phase.

"Coupled" as used in this specification refers to the direct contact as well as the disposition of a linker gene or protein between two or more types of genes or proteins. It also includes the coupling through covalent or non-covalent bonds.

"Target protein" as used in this specification refers to any protein to be purified. An example of a target protein can be an antibody.

A "tagged target protein" refers herein to a target protein covalently bonded to a peptide tag.

A "fusion protein" refers herein to any combination of two or more separate polypeptides. An example of the fusion protein may be a protein created through the joining of two or more nucleic acids that originally encoded separate polypeptides. A tagged target protein can be a fusion protein in which the nucleic acid encoding the peptide tag is ligated to the nucleic acid encoding the target protein to obtain a nucleic acid encoding the fusion protein.

The method disclosed herein uses the high binding specificity between a protease and a specific inhibitor of the protease to purify a target protein.

In one aspect, the method includes contacting a sample containing a tagged target protein to a support having immobilized thereon a protease inhibitor, wherein the tagged target protein is a target protein covalently bound to a peptide tag and the peptide tag has specific binding affinity for the protease inhibitor, and wherein the contact is under conditions such that the protease inhibitor binds to the peptide tag; removing components of the sample that are not bound; and then removing the target protein from the support. The sample may be a cell lysate, a cell culture, the supernatant of a cell culture, or a biological fluid containing the target protein. In a further aspect, the support having immobilized thereon a protease inhibitor is configured as an affinity chromatography column.

In the method and column for the purification of proteins disclosed herein, a disposable column may be used. The disposable column may have a diameter of about 0.1 to about 1.0mm, about 0.3 to about 0.7mm, or about 0.5mm. The column may be placed in a glass test tube of about 16 x 125 mm. In an embodiment, a disposable column having the diameter of 0.5mm is placed in a 16 x 125 mm glass test tube. Gel may be packed into the column according to any one of methods known and widely used in the art. In an embodiment, a sufficient volume of degassed buffer/water is added to the column to fill it up to the reservoir (wide-mouth) portion, then any air bubbles is eliminated in the column. After that, gel may be packed into the column with degassed 5.0% gel slurry, and degassed buffer solution (or water) to room temperature. Sufficient volume of degassed gel slurry may be added to obtain the desired settled gel volume. Gel may settle down in the column for at least 30 minutes. The packed column may be stored and used at 4°C.

Removing the target protein from the support can comprise eluting the tagged target protein from the support; followed by separating the peptide tag from the tagged target protein to obtain the target protein. Eluting the tagged target protein can be conducted by elution at a pH that lowers the binding affinity between the peptide tag and the protease inhibitor such that the tagged target protein is removed from the support. Separating the peptide tag from the tagged target protein can be achieved by treating the eluted tagged target protein with a protease, for example TEV protease, papain, or pepsin.

Alternatively, the target protein can be removed from the column by enzymatically cleaving the bound peptide tag from the target protein on the column, leaving the peptide tag bound on the column and the target protein free to be eluted from the column. Examples of proteases include TEV protease, papain, or pepsin.

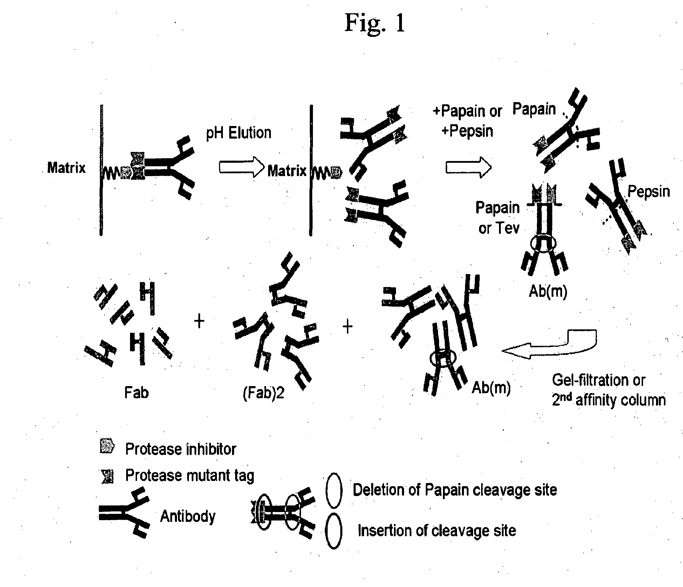

Fig. 1 shows a schematic diagram of an embodiment of the protein purification method disclosed herein. In the embodiment shown, the target protein is an antibody including a peptide tag covalently added to the C-terminus of the immunoglobulin heavy chain. In the embodiment shown, a protease inhibitor which specifically binds the peptide tag is immobilized on the support to obtain an affinity chromatography medium. If a sample containing the target protein flows through the affinity chromatography column containing the affinity chromatography medium, the target protein will be retained on the column due to the specific binding between the peptide tag and the protease inhibitor and other components of the sample will not be retained on the column. Then the bound target protein can be removed from the column.

As shown in Fig.1, the bound tagged target protein can be removed from the column by elution at conditions at which the peptide tag will no longer bind the protease inhibitor, e.g., elution in a buffer with a lower pH. The eluted target protein can then be separated from the peptide tag. For example, as shown in Fig. 1, F(ab)2 or Fab fragments can be obtained by treating the tagged target antibody with an enzyme, such as pepsin or papain. In addition, if the target antibody heavy chain is mutated to destroy the natural papain cleavage site of the antibody, and to insert a new papain cleavage site between the peptide tag and the target antibody, then the peptide tag can be removed from the tagged target antibody by treating the fusion protein released from the column with papain. Peptide tags which may be used in the method disclosed herein include peptide tags derived from a mutant protease from any of the following classes of proteases: serine proteases, threonine proteases, cysteine proteases, aspartic acid proteases, metalloproteases and glutamic acid proteases. Specifically, peptide tags include peptide tags derived from mutants of papain, trypsin, pepsin, chymotrypsin, rennin, cathepsin D, thermolysin, elastase, plasmin, thrombin, urokinase, or collagenase.

Peptide tags can be prepared by any method known in the art. Mutation can occur only at the minimum level necessary to inhibit the hydrolysis activity. For example, a protease mutant to be used as a peptide tag can be prepared by substituting a single nucleotide or a single amino acid. In this regard, site-direct mutagenesis, a method widely known in the art, can be used. In some embodiments, the entire proteolytic active site is deleted from the peptide tag. The deletion can be made using any method known in the art.

Protease inhibitors that can be used in the method or the affinity chromatography medium disclosed herein include, ecotin, papaya Kunitz-type trypsin inhibitor, pepstatin A, leupeptin, Gly-Gly-Tyr-Arg peptide, a2-macroglobulin, a2-antiplasmin, antithrombin III human, a1-antitrypsin, bdellin, pepsinostreptin, chymostatin, phosphoramidon, isoamylphosphonyl-Gly-L-Pro-L-Ala and dipotassium 2(R)-2-mercaptomethyl-4-methylperitanoyl-beta-(2-naphthyl)-Ala-Ala amide. Specifically, the protease inhibitor can be any of those listed in Table 1 or in Table 2 below.

**[Table 1]**

| Protease Inhibitors | Example Commercial Source |
|---|---|
| a2-Antiplasmin | Product Code A0914 (Sigma) |
| Aprotinin | Product Code A1153 (Sigma) |
| Antithrombin III, Human | Product Code A2221 (Sigma) |
| a1-Antitrypsin | Product Code A6150 (Sigma) |
| Antipain | Product Code A6191 (Sigma) |
| Antithrombin III, Bovine | Product Code A9141 (Sigma) |
| Bdellin | Product Code B3906 (Sigma) |
| Ecotin | Product Code B3910 (Sigma), or manual purification |
| Leupeptin | Product Code B3912, L2023 (Sigma) |
| a2-Macroglobulin | Product Code B3913, M6159 (Sigma) |
| Na-p-Tosyl-L-lysine chloromethyl ketone hydrochloride | Product Code B3918, T7254 (Sigma) |
| Trypsin-chymotrypsin inhibitor | Product Code B3923 (Sigma) |
| Chymostatin | Product Code C7268 (Sigma) |
| Cystatin | Product Code C8917 (Sigma) |
| E-64 | Product Code E3132 (Sigma) |
| Ebselen | Product Code E3520 (Sigma) |
| Gly-Gly-Tyr-Arg | Product Code G5386 (Sigma) |
| Na-p-Tosyl-L-phenylalanine chloromethyl ketone hydrochloride | Product Code A0917 (Sigma) |
| Trypsin Inhibitor from Glycine max (soybean) | Product Code T1021, T9767 (Sigma) |
| Papaya Kunitz-type trypsin inhibitor | Manual purification |

Examples of combinations of a protease and a protease inhibitor that can be used in the method disclosed herein are provided in Table 2 below.

**[Table 2]**

| Protease class | Representative Protease | Protease Inhibitor |
|---|---|---|
| Cysteine protease | Papain | Leupeptin |
| | | Gly-Gly-Tyr-Arg |
| | | a2-Macroglobulin |
| Serine protease | Trypsin | Ecotin |
| | | Papaya Kunitz-type trypsin inhibitor |
| | | a2-Antiplasmin |
| | | Antithrombin III, Human |
| | | a1-Antitrypsin |
| | | Bdellin |
| | | Leupeptin |
| | | a2-Macroglobulin |
| Aspartic protease | Pepsin | Pepstatin A Pepsinostreptin |
| | Rennin | Pepstatin A |
| Aspartic protease | Cathepsin D | Pepstatin A |
| | | Chymostatin |
| Metalloprotease | Thermolysin | Phosphoramidon |
| | | a2-Macroglobulin |
| | Collagenase | Ecotin Isoamylptiosphonyl)-Gly-L-Pro-L-Ala, dipotassium (2R)-2-mecaptomethyl -4-methylpentanoyl - beta (2-naphthyl)-Ala-Ala amide |

In an embodiment of the method or column disclosed herein, the protease inhibitor is ecotin, a serine protease inhibitor from E. coli which inhibits trypsin and other pancreatic proteases. Ecotin remains stable for at least thirty minutes at 100°C, pH 1.0. The molecular weight of ecotin is 18kD, and its isoelectric point (Pi) is 6.1. Ecotin is not digested by trypsin, chymotrypsin, pancreatic elastase, rat mast cell chymase, or human serosal urokinase. Further, ecotin does not inhibit human pulmonary tryptase, kallikrein, papain, pepsin, staphylococcus aureus V8 protease, subtilisin, or thermolysin. Also, it does not inhibit any of the eight soluble endoproteases recently isolated from *E*. *coli* (i.e., Do, Re, Mi, Fa, So, La Ci and Pi), chymotrypsin-like esterase (protease I) or trypsin-like esterases (protease II). In an embodiment of the method in which the protease inhibitor is ecotin, the protease is trypsin. Trypsin is a serine protease found in the digestive system which breaks down protein. For example, Trypsin breaks down casein in milk. Trypsin mostly cleaves peptide chains at the carboxyl side of the amino acids lysine and arginine, except when either is followed by proline. Trypsin exists in large quantities in the pancreas, and can be purified without difficulty.

In another embodiment of the method or column disclosed herein, the protease inhibitor is papaya Kunitz-type trypsin inhibitor, a member of the class of papaya proteinase inhibitors (PPI) derived from *Carica papaya.* Papaya Kunitz-type trypsin inhibitor inhibits bovine trypsin in a molar ratio of 1:1. Papaya Kunitz-type trypsin inhibitor is active as an inhibitor over a very wide range of pH (1.5 to 11.0) and at temperatures as high as 80°C. It is also stable in high concentrations of strong chemical denaturants (e.g., 5.5 M guanidine hydrochloride). Papaya Kunitz-type trypsin inhibitor shows outstanding resistance to degradation by pepsin. In some embodiments of the method in which the protease inhibitor is a papaya Kunitz-type trypsin inhibitor, the protease is trypsin.

The nucleic acid encoding a target protein may be engineered to include a peptide tag, i.e., the gene can be engineered to encode a fusion protein including the target protein and a peptide tag. The gene encoding the fusion protein is then introduced into a vector, and then the vector is used to transform a suitable host cell. As noted before, if the target protein includes a sequence susceptible to the protease from which the peptide tag is derived, it is important to prevent the proteolysis of the target protein by the protease of the peptide tag. This can be prevented by, for example, mutagenesis of the gene(s) of the protease in the region of the proteolytic active site. Mutagenesis, formation of the recombinant nucleic acid encoding the fusion protein (tagged target protein), formation of the recombinant expression vector, and transformation of the host cell may be accomplished by any of the methods known in the relevant field of technology.

The term "vector" refers to a nucleic acid molecule capable of transporting another nucleic acid to which it has been linked and directing expression of a nucleic acid encoding a target protein operably linked thereto. Moreover, the vector may have an appropriate marker to screen a transformed host cell. Available vectors may include bacteria, plasmids, phages, cosmids, episomes, viruses, and insertable DNA fragments (fragments able to be inserted into a host cell genome by homologous recombination).

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., that the coding sequence is under the transcriptional control of the promoter) or a ribosome binding site is operably linked to a coding sequence if it is positioned so as to facilitate translation. Coding sequences can be operably linked to regulatory sequences in a sense or antisense orientation.

The vector may be introduced into a host cell, and produce a fusion protein or peptide encoded by the nucleic acids mentioned above. In some cases, the vector may contain a promoter recognized by the host cell. By "promoter" is meant minimal sequence sufficient to direct transcription. Also included are those promoter elements which are sufficient to render promoter-dependent gene expression controllable for cell-type specific or inducible by external signals or agents; such elements may be located in the 5' or 3' regions of the gene. Both constitutive and inducible promoters are included. The promoter sequence may originate from a prokaryote, a eukaryote, or a virus. Selecting a host cell-compatible promoter with a suitable promoter activity for the desired level of expression of a nucleic acid in the host cellt is within the skill of the ordinary artisan.

The vector may have an additional expression control sequence. "Control sequences" refers to DNA sequences necessary for the expression of an operably linked coding sequence in a particular host organism. The control sequence may be a Shine-Dalgarno sequence. For example, the Shine-Dalgarno sequence can be from the replicase gene of phage MS-2 or from the cII gene of bacteriophage 1. The transformation of a host may be accomplished by any one of a variety of techniques well known in the art.

The protease inhibitor affinity chromatography medium and column disclosed herein can be prepared by irrimobilizing a protease inhibitor through reductive amination to agarose beads activated by aldehyde and sodium cyanoborohydride (NaBH₃CN).

The protease inhibitor affinity column disclosed herein may be prepared as follows:
1. Preparation of the support:
   A support to which the coupling of protease inhibitors may occur is prepared. Agarose can be used as such a support. For example, freeze-dried agarose beads suspended in solvent can be used. An example of freeze-dried agarose beads can be cyanogen bromide (CNBr)-activated Sepharose 4B (GE Health Care), obtained by freeze-drying CNBr-activated Sepharose 4B in the presence of additives. Before coupling the protease inhibitor to the Sepharaose 4B, the additives are removed by washing the CNBr-activated Sepharose 4B at a low pH to preserve the activity of the reactive group. In this regard, the pH may be 3 or lower as higher pH may induce hydrolysis. Freeze-dried agarose beads are suspended in a solvent, for example 1mM HCl. Normally, 1g of freeze-dried CNBr-activated Sepharose 4B beads suspended in the solvent will give about 3.5ml final volume of the support after swelling. The support should be further washed when the support swells, for example for 15 minutes with 1mM HCl on a sintered glass filter of porosity G3.
2. Coupling the protease inhibitors
   This step involves the coupling the protease inhibitor with the support provided in Section 1 above. First, the protease inhibitor is dissolved in a coupling solution. About 3 to 7ml of such coupling solution can be used per initial gram of freeze-dried Sepharose powder. Also, about 5 to 10 mg of protease inhibitor can be used per 1ml of swollen support. If the molecular weight of the protease inhibitor is small (5kD or less), it may be added in concentrations of 1 to 10 µmol per ml of support. An example of a coupling solution is 0.1M NaHCO₃ (pH 8.3)/0.5M NaCl. The support suspension prepared in accordance with Section 1 above is added to the coupling solution containing the protease inhibitor and stirred. Any method of gentle stirring may be used without limitation, e.g. rotating the mixture end-overend.

In an embodiment of the method of purifying a target protein, eluting the bound target protein can occur in any suitable solvent that permits elution of the target protein from the protease inhibitor affinity chromatography medium. In some embodiments, elution can be conducted by means of pH elution. For example, target proteins coupled with peptide tags may be eluted by adjusting the pH to be within the range of 2 to 5. If ecotin is used as the protease inhibitor, for example, the elution may be conducted at pH 2 to 3 as ecotin shows very high stability and high affinity to its complementary peptide tags even in strong acid. The lower the pH, the easier and more efficient the elution will be, resulting in higher yield and higher purity of proteins. If papaya Kunitz-type trypsin inhibitor is used, it is very stable in acid but its affinity to its complementary peptide tags is about 1/100 lower compared to that of ecotin, so the elution will be possible at pH 3 to 4. As shown in the foregoing examples, the affinity between peptide tags and protease inhibitors vary and different elution pH values may be used for different combinations of protease inhibitor affinity medium and complementary peptide tag. Another method of separating target proteins bound to peptide tags from the column involves the use of active proteases. The active proteases selected for use in such a method should be those which are not capable of degrading the protease inhibitors on the column, or rendering the target protein inactive. For example, as discussed above, when such a method is used for a target antibody, a complete antibody molecule or antibody fragments (Fab, F(ab)2) can be eluted.

Separating the peptide tag from the target protein, although not particularly limited, can be conducted by treating with enzymes. For example, in the case of an antibody as the target protein, if the tagged antibody is treated with an enzyme such as papain or pepsin, F(ab)2 or Fab will be obtained, as discussed previously. A tagged target antibody which can provide a complete immunoglobulin upon proteolysis with papain may be obtained by mutating the papain cleavage site of the immunoglobulin heavy chain gene, inserting a papain cleaving site between the nucleic acid encoding the peptide tag and the immunoglobulin heavy chain gene, and treating the tagged antibodies so obtained with papain. This is shown in the right lower part of Fig. 1. Alternatively, TEV protease cleavage sites can be inserted between the peptide tag and the protein. The peptide tag may be separated from the protein by digesting the fusion protein with TEV protease.

Hereinafter, the method and chromatography disclosed herein will be more specifically described with reference to the following examples. However, these examples are for illustrative purposes only.

### □ Mode for Invention □

### EXAMPLES

### Example 1. Purification of ecotin

### 1. Preparation of ecotin

Recombinant ecotin of Sequence ID No. 1 is overexpressed in *Escherichia coli.* The expressed cells are collected by centrifugation at 5,000 x g for 15 minutes. The cells are distrupted by sonicator and the crude extracts are obtained by centrifugation at 12,000 x g for 30 minutes. The crude extracts are treated by adding 1 M HCl and are adjusted to pH 3. The ecotin was obtained in supernatants by centrifugation at 12,000 x g for 10 minutes. After incubating the recombinant ecotin for 20 minutes at 4°C, the pH is readjusted to 7.8 by adding 1M Tris base, and heated at 100°C for 20 minutes. For each step, insoluble materials are removed by centrifugation at 12,000 x g for 10 minutes to obtain supernatants, and solid ammonium sulfate is added to the supernatants to 80% (w/v) saturation to obtain fractionated proteins at 10% intervals.

The precipitated proteins are dialyzed against 10mM Tris-HCl (pH7.8) containing 6mM MgCl₂ and loaded onto an anion exchange column equilibrated with the same buffer. Protein that does not bind to the column is collected, purified using a trypsin affinity column and stored at 4°C.

### 2. Gel Electrophoresis

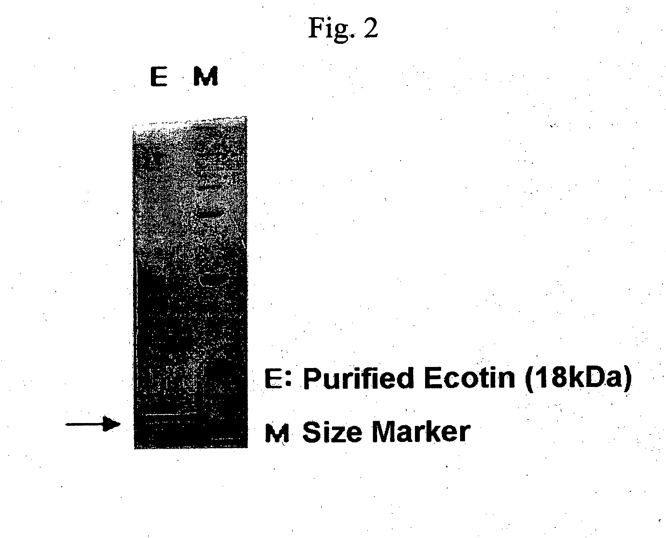

Purity of ecotin obtained in accordance with the method described in Section 1 above was determined by electrophoresis carried out using 10-20% (w/v) polyacrylamide gradient slab gels containing 0.1% (w/v) sodium dodecyl sulfate (SDS) as described by Laemmli (Laemmli, U.K. (1970) Nature 227, 680-685). The results of electrophoresis are shown in Fig. 2. Purity of the ecotin is determined to be 95% or higher.

### 3. Measurements of Activity of Ecotin

In order to test whether the ecotin obtained as described above shows protease inhibitor activity, the cleavage of fluorogenic peptides is assayed as described by Woo (Woo, K.M., Chung, W. J., Ha, D.B., Goldberg, A. L., and Chung, C. H. (1989) J. Biol Chem. 264, 2088-2091). N-benzyloxycarbony-Ala-Arg-Arg-4-methoxy-β-naphthylamide, N-succinyl (Suc)-Leu-Leu-Val-Tyr-7-amido-4-methyl coumarin (AMC) and Suc-Ala-Ala-Ala-AMC are used as the substrates for trypsin, chymotrypsin and elastase, respectively. 0.1 ml of a reaction mixture containing appropriate amounts of ecotin, and 10 ng of trypsin, 2ng of chymotrypsin or 30 ng of elastase in 100 mM Tris-HCl buffer (pH 8) is incubated for 30 min at room temperature prior to the addition of the peptide substrates (0.1mM). When assaying trypsin and chymotrypsin, 20mM CaCl₂ is also included. After the incubation, 0.9ml of 0.1x sodium borate (pH 9.1) is added to the reaction mixture, and then the reaction mixture is heated for 6 minutes in a boiling water bath. Fluorescence is determined at 310 nm (excitation) and 410 nm (emission) for 4-methoxy-p-naphthylamide and at 380 nm and 440 nm for AMC. Proteins are assayed a described by Bradford (Bradford, M. M. (1976) Anal. Biochem. 72, 248-254) using bovine serum albumin as a standard or by absorbance at 280nm for those proteins whose extinction coefficients are known. The ecotin obtained in Section 1 above shows 100% inhibition of the activities of trypsin, chymotrypsin and elastase.

### Example 2 Preparation of the ecotin affinity column

A disposable column(diameter 0.5mm) is used for purification of antibody. The column is placed in a 16 x 125 mm glass test tube (in a test tube rack). A sufficient volume of degassed buffer/water is added to the column to fill it up to the reservoir (wide-mouth) portion, then any air bubbles is eliminated in the column. Gel is packed into a Column with degassed 50% gel slurry, and degassed buffer solution (or water) to room temperature. Sufficient volume of degassed gel slurry is added to obtain the desired settled gel volume. Gel settles down in the column for at least 30 minutes. The packed column is stored and used at 4°C.

Purified ecotin is immobilized by reductive amination using CNBr-activated Sepharos 4B (GE Health Care) in this example.

### 1. Preparing the chromatography medium

CNBr-activated Sepharose 4B (GE Health Care) is suspended in 1mM HCl and left for 30 minutes or longer until the gel is swollen. 1g of freeze-dried Sepharose power gives about 3.5ml final volume of medium. When the support settles immediately after suspension, the supernatant is discarded, and this process is repeated to separate broken medium fragments, etc. Then the support is washed with 15 support (gel) volumes of cold 1mM HCl. The support is then washed with the coupling buffer of 0.1M NaHCO₃ (pH 8.3)/0.5 NaCl in order to replace the support buffer with the coupling buffer.

### 2. Coupling the ecotin

The ecotin obtained through the process of Example 1 is dissolved in coupling buffer, 0.1 M NaHCO₃ (pH 8.3) containing 0.5M NaCl. About 7 ml of coupling buffer is used per 1 gram of freeze-dried Sepharose powder. About 10 mg of ecotin is used per ml support. To the coupling solution containing ecotin, the sepharose suspension prepared in Section 1 above is added, and the mixture is stirred for 3 to 4 hours at room temperature overnight at 4°C. The reaction is stopped by adding a stop buffer (0.1M ethanolamine) to the coupled agarose. Then excess ecotin is washed away using at least 5 medium (gel) volumes of coupling buffer. The chromatography medium is transferred to 1 M ethanolamine, pH 8.0 and let stand for 2 to 4 hours. Then the chromatography medium is washed through eight cycles of alternating pH. The volume of each of the buffers used for washing is five times the volume of the medium. Each cycles consists of a wash with 50mM glycine, 1M NaCl (pH 3.5) followed by a wash with 50mM Tris-HCl (pH 8.0)/1M NaCl. Then the chromatography medium is washed again with phosphate buffered saline (PBS); of 10 medium volumes.

### Example 3: Purification of trypsin using ecotin affinity chromatography

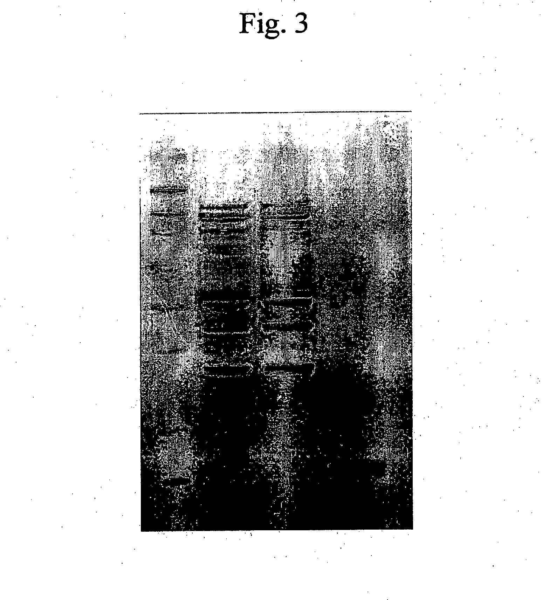

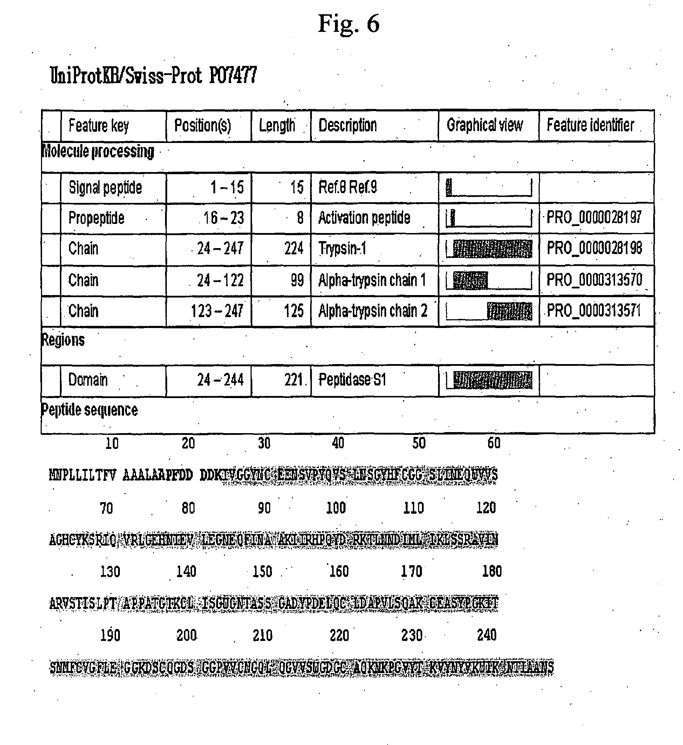

Human cationic trypsinogen (PRSS1) gene (SEQ ID NO: 2) is cloned. The gene for wild type human PRSS1 (HGNC:9475; UniProtKB/Swiss-Prot P07477)(Fig. 6, SEQ ID NO: 13) is subjected to site-directed mutagenesis in the proteolysis activity site in order to obtain a mutant PRESS that lacks the proteolysis anctivity and cannot hydrolyze the anti-EGFR antibodies (SEQ ID NO: 2). In particular, a site-directed mutagenesis kit (Stratagen, #200524-5, site-Directed Mutagenesis kit) is used to mutate the codon of the 200^{th} serine amino acid to that for alanine. The obtained mutant PRSS1 gene (SEQ ID NO: 2) is cloned into a pET21 b(Novagen) vector and expressed in *E. coli* BL21(DE3) grown in YT medium. When O.D. 600 of 0.6 is reached, 1mM Isopropyl-β-D-Thiogalactopyranoside IPTG) is added to the medium and the medium is further cultured at 37°C for 4 hours. The cultured cells are lysed using ultrasonic waves in a buffer (50 mM Tris-HCl, pH 8.0, 0.2 M NaCl and 6M Guanidine-HCl) and centrifuged at 10000g to obtain a supernatant. The supernatant is dialyzed with a buffer (50 mM Tris-HCl, pH 8.0, 0.2 M NaCl and 0.9M Guanidine-HCl) to refold denatured protein. The sample obtained above is applied to the 1-mL ecotin column prepared in accordance with Example 2. The column is washed with the same buffer and eluted with 50 mM HCl to recover purified trypsinogen. The trypsinogen obtained above is subjected to electrophoresis, as described in Example 1. The results are shown in Fig. 3. Lane 1 (leftmost lane) shows size markers, Lane 2 (2^{nd} from left) shows the crude protein extract (not purified), Lane 3 (3^{rd} from left) is the flow through (not bonded to ecotin), Lane 4 (rightmost lane) is purified trypsin mutant only.

### Example 4: Purification of anti-ecotin antibodies using ecotin affinity chromatography

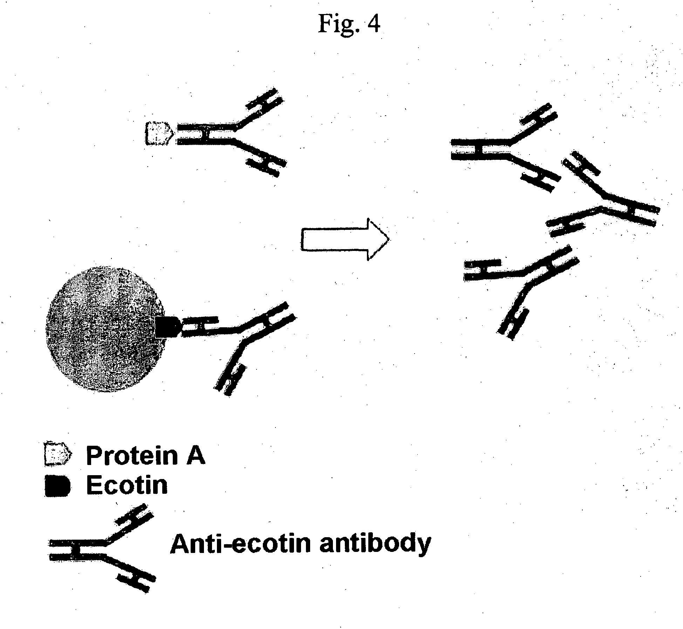

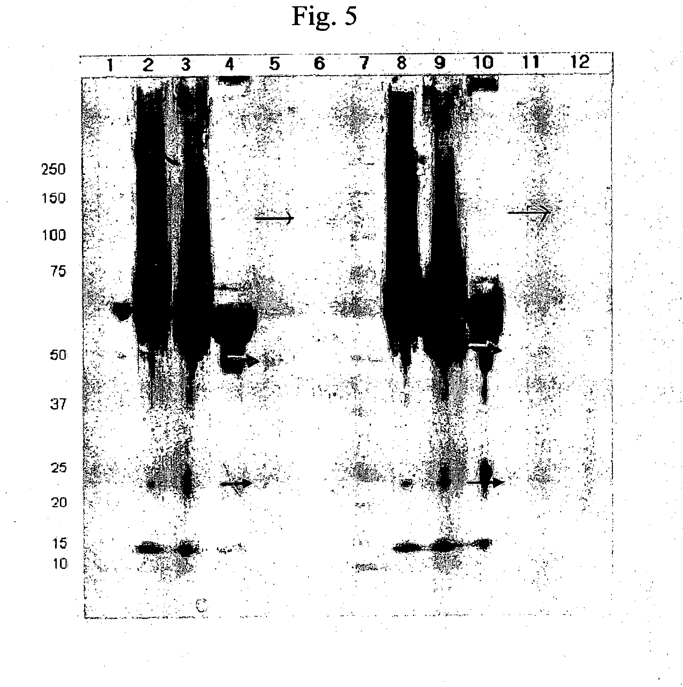

200 ug of ecotin obtained in Example 1 is dissolved in 10mM Tris-HCl (pH 7.8) and then subcutaneously injected into a New Zealand White rabbit (two-month aged male) to generate anti-ecotin antibodies. After antibody generation, the rabbit's serum is collected. A sample of the serum is applied directly to a Protein A sepharose CL-4B column (GE Healthcare) or to the ecotin column prepared in Example 2, respectively, using a loading buffer(50 mM Tris-HCl, pH 8.0, and 0.2. M NaCl). E ach column is then washed with a buffer (50 mM Tris-HCl, pH 8.0, and 0.5M NaCl) and then eluted with a glycine buffer (50 mM Glycine-HCl, pH 2.5). Fig. 4 is a diagram schematically showing the purification of anti-ecotin antibodies using a Protein A affinity column or an ecotin affinity column, respectively. Anti-ecotin antibodies purified by Protein A affinity column chromatography or by ecotin affinity column chromatography were analyzed by protein gel electrophoresis, as described in Example 1. Fig. 5 is a photograph of the results of the electrophoresis.

In Fig. 5, Lane 1 and Lane 7 are size markers, Lanes 2 to 6 are results from Protein A column chromatography while Lanes 8 to 12 are results of the ecotin column chromatography. In particular, Lanes 2 and 8 are crude extracts (serum not applied to the column), Lanes 3 and 9 are flow-through extracts (applied but non-bonded to the column), Lanes 4 and 10 are extracts obtained through washing with a buffer including 0.5M NaCl, Lanes 5 and 11 are results of the elution solution to which dithiothreitol(DTT) is added and Lanes 6 and 12 results of the elution solution to which DTT is not added.

### Example 5: Expression of anti-EGFR-Trypsin monoclonal antibody in HEK293 cells

In this example, anti-EGFR-Trypsin monoclonal antibodies, i.e., monoclonal EGFR antibodies tagged with trypsin tags, are transiently over-expressed in HEK293 cells (ATCC. CRL-1573) and characterized. Table 3 below shows the amino acid sequences of the Complementarity Determining Regions (CDRs) of the anti-EGFR antibodies used in this example.

**[Table 3]**

| **CDR** | **Amino Acid Sequences** | |
|---|---|---|
| | Heavy Chain | Light Chain |
| CDR 1 | DYGMS (SEQ ID NO: 7) | TGTSSDVGGYNYVS (SEQ ID NO: 10) |
| CDR 2 | GINWNGGSTGYADSVKG (SEQ ID NO: 8) | DVNRRPS (SEQ ID NO: 11) |
| CDR 3 | DYWGSLDY (SEQ ID NO: 9) | SSYVSTNTYV (SEQ ID NO: 12) |

The monoclonal anti-EGFR antibodies are obtained using the phage library display library panning with the EGFR protein (Sigma E3641) as the target antigen. The amino-acid sequences of the Heavy and Light chain variable region about anti-EGFR antibodies are prepared and then the variable regions of Heavy chain sequences on the modified pcDNA3.3 heavy chain vector are replaced with a human full IgG1 Heavy chain antibody backbone. The variable regions of Light chain sequences on the modified pcDNA3.3 Light chain vector are replaced with a human full IgG1 Light chain antibody backbone.

Further, the fragment crystallizable region (Fc) of the monoclonal anti-EGFR-antibody has the sequence of SEQ ID NO: 5. SEQ ID NO: 5 is the sequence of Hinge, CH2 and CH3 only in the heavy chain.

The trypsin tags are obtained from mutant human PRSS1 (HGNC:9475)(SEQ ID NO: 2). The gene for wild type human PRSS1 (HGNC:9475; UniProtKB/Swiss-Prot P07477)(Fig. 6, SEQ ID NO: 13) is subjected to site-directed mutagenesis in the proteolysis activity site in order to obtain a mutant PRSS1 that lacks the proteolysis anctivity and cannot hydrolyze the anti-EGFR antibodies. In particular, a site-directed mutagenesis kit (Stratagen, #200524-5, site-Directed Mutagenesis kit) is used to mutate the codon of the 200^{th} serine amino acid to that for alanine.

The mutated PRSS1 gene is amplified by PCR using the followingPCR primers: XhoI-TEV-Forward (SEQ ID NO: 3, 5'-GAG-CTCGAG-GAA AAC CTG TAT TTT CAG GGA TCC- ATCGTTGGGGGCTACAACTGTGAGGAG) and XhoI-TAA-R (SEQ ID NO: 4, 5'-GAG-CTCGAG-TTA GCT GTT GGC AGC TAT GGT GTT CTT A). Using these primers, the nucleic acid encoding the trypsin domain (24-244aa, SEQ ID NO: 2), which lacks the signal peptide (1-l5aa) and the propeptide (16-23aa), is amplified from the mutant PRSS1 gene and is used as the nucleic acid encoding the trypsin tag for ligation to the nucleic acid encoding the heavy chain of the anti-EGFR antibody. The cDNA of the anti-EGFR antibody heavy chain is SEQ ID NO: 14. Fig. 6 is a reproduction of part of Swiss-Prot entry P07477, for wild type human cationic trypsinogen (PRSS1) showing the polypeptide sequence (SEQ ID NO:13) and structural features of the polypeptide sequence. According to the Fig. 6, 1-15aa of PRSS1 protein is the signal peptide and 16-23 aa is the propeptide.

The trypsin tag nucleic acid obtained above is cleaved by the restriction enzyme, XhoI, and ligated into the XhoI-site (positions in SEQ ID NOs:14 and 16) of the carboxyl-terminal end of the nucleic acid encoding the heavy chain of the anti-EGFR antibody using T4-DNA lygase (NEB, M0202L). The stop codon (TGA) in positions 697~699 of the cDNA encoding Fc of the heavy chain of the anti-EGFR antibody (SEQ ID NO: 5) is deleted using a site-directed mutagenesis kit (Stratagen, #200524-5). A Tev protease cleavage site (5'-GAA AAC CTG TAT TTT CAG GGA TCC-3', SEQ ID NO: 6) is also inserted between the antibody cDNA which lacks the stop codon and the trypsin tag nucleic acid prepared above.

The tagged anti-EGFR heavy chain construct described above (SEQ ID NO: 16) is inserted into a vector, using the pcDNA™3.3-TOPO^{®} TA Cloning^{®} Kit (Invitrogen). Also, the gene encoding the light chain of the anti-EGFR antibody (SEQ ID NO: 15) is also inserted into a vector using the pcDNA™3.3-TOPO^{®} TA Cloning^{®} Kit (Invitrogen). 12µg of each of the heavy chain vector and the light chain vector obtained above is prepared. The total 24 ug amount of the two vectors is transfected into HEK293 cells using lipofectamine 2000 (Invitrogen). The transfected HEK293 cells are cultured in Dulbecco's modified Eagle's medium (DMEM) (Gibco cat no 11995) containing 100U/ml of the antibiotics penicillin and 100ug/ml of streptomycin and 10% fetal bovine serum (FBS) at 37°C and 5% CO₂ for 3 days. Expressed heavy chains and light chains are separately secreted into the medium, where the heavy chains and light chains can associate with each other to form the antibody complex. After that, the supernatant of the cell culture is collected and applied to the ecotin column prepared in Example 2. The purification process is as follows:

The ecotin affinity column prepared as above is set up through any well-nown method. The pH of the cell culture supernatant is adjusted by adding 1/10 volume of 1.0M Tris-HCl (pH8.0). Then, the cell culture supernatant is passed through the ecotin affinity column. The column is washed with 10 column volumes of 50mM Tris - HCl (pH8.0)/0.5M NaCl. It is then washed with 10 column volumes of 50mM sodium acetate (pH5.6)/0.5M NaCl. Trypsin-tagged monoclonal antibodies are eluted from the column with a glycine buffer (50mM Glycine-HCl, pH 2.5). The eluent is then neutralized by adding 1 M Tris-HCl (pH9.0).

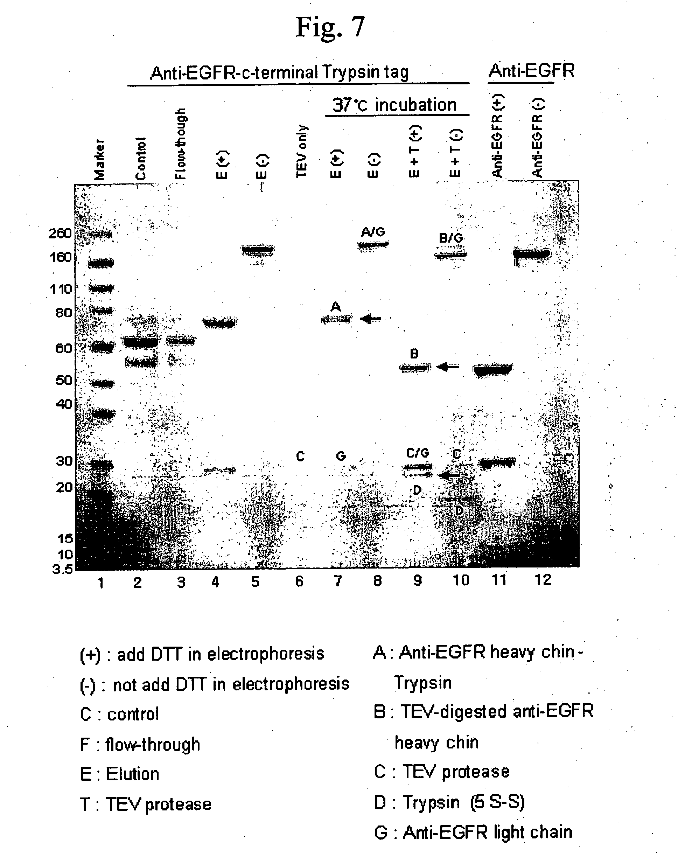

Various samples from the purification process were analyzed by protein gel electrophoresis, as described in Example 1. The results are shown in Fig. 7, which is a photograph of the electrophoresis gel. The results in Fig. 7 confirm the expression in the HEK293 cells of the recombinant antibody.

In Fig. 7, Lane 1 has size markers. Lanes 2 to 10 are results for electrophoresis of solutions from the ecotin affinity purification of the anti-EGFR antibody which has its heavy chain tagged with the mutant trypsin and Lanes 11 and 12 contain anti-EGFR antibodies without trypsin tags, subjected to electrophoresis in the presence or absence of DTT, respectively. Lane 2 is a control lane showing the crude cell culture supernatant before applying to the column. Lane 3 is the column flow-though (F), i.e, proteins which are not bonded to the ecotin after applying to the column. Lane 4 is the eluent (E(+)), the solution eluted from the column to which DTT is added in electrophoresis. Lane 5 is the eluent solution (E)to which DTT is not added (-) in electrophoresis. Lane 6 is the TEV protease only; the TEV protease band is labeled C within the gel photo. Lanes 7 to 10 show the results for the Eluent (E) in the presence (+) or absence (-) of DTT and the presence or absence of the TEV protease (T) incubated at 37°C. Lane 7 is E(+) (identical to Lane 4), Lane 8 is E(-)(identical to Lane 5), Lane 9 is E+T(+), and Lane 10 is E+T(-).

In the annotations within the gel photo of Fig. 7, A is the anti-EGFR heavy chain-trypsin fused protein band, B is the TEV protease-digested anti-EGFR heavy chain band, C is the TEV protease band, D is the cleaved trypsin tag band (a single construct having five S-S bonds), and G is the anti-EGFR light chain band.

### Example 6: Purification of monoclonal antibodies using ecotin affinity chromatography

In this example, the anti-EGFR-Trypsin monoclonal antibodies described in Example 5 are over-expressed in CHO-DG44 (DHFR⁻) cell (Invitrogen. Cat.no. 12613-014).

As described in Example 5, the anti-EGFR heavy chain gene tagged with the mutant trypsin is inserted into a vector using a pcDNA™3.3-TOPO^{®} TA Cloning^{®} Kit (Invitrogen). Also, the gene encoding the light chain of anti-EGFR antibodies is inserted into a vector using the pcDNA™3.3-TOPO^{®} TA Cloning^{®} Kit (Invitrogen). In the current experiments, 18.75µg of each of the heavy chain vector and the light chain vector obtained above is prepared.

Before transfecting these vectors, CHO-DG44 cells are sub-cultured two times to 6 x 10⁵ cells/ml at 37°C, 8% CO₂ under the condition of 130rpm once before 48 hours and once before 24 hours, respectively, in CD DG44 medium (Gibco. Cat no. 12610) to which 8mM L-glutamine and 0.18% PLURONIC F-68 are added. Cells exhibiting surviving rates of 95% or higher are adjusted to 3 x 10⁷ cells, put into 125ml flask and CD DG44 medium is added to the flask to adjust the final volume to 30ml. The 18.75 µg of each of the heavy chain vector and the light chain vector, and 37.5µl FreeStyle MAX reagent (Invitrogen, 16447100) are mixed slowly using OptiPro SFM (Invitrogen, serum free medium) to adjust the final volume to 1.2ml. Then, the mixture is incubated at room temperature for 10 minutes. The plasmid-lipid complex so obtained is slowly added to the flask containing CHO-DG44(DHFR⁻) cells and then the cells are cultured in a CD DG44 medium supplemented with 500 µg/ml Geneticin for 5 days. Then, the culture medium is collected and subjected to the ecotin affinity chromatography medium prepared in Example 2, as follows.

The ecotin affinity column prepared as above is set up through any well-nown method. The supernatant of the cell culture obtained above contains about 4µg/ml of antibodies. The pH of the cell culture supernatant is adjusted by adding 1/10 volume of 1.0 M Tris-HCl (pH8.0). Then, the cell culture supernatant is passed through the ecotin affinity column. The column is washed with 10 column volumes of 50 mM Tris HCl (pH8.0)/0.5 M NaCl. It is then washed with 10 column volumes of 50 mM sodium acetate (pH5.6)/0.5 M NaCl. Trypsin-tagged monoclonal antibodies are eluted from the ecotin affinity chromatography medium with a glycine buffer (50 mM Glycine-HCl, pH 2.5) and neutralized by adding 1 M Tris-HCl (pH9.0).

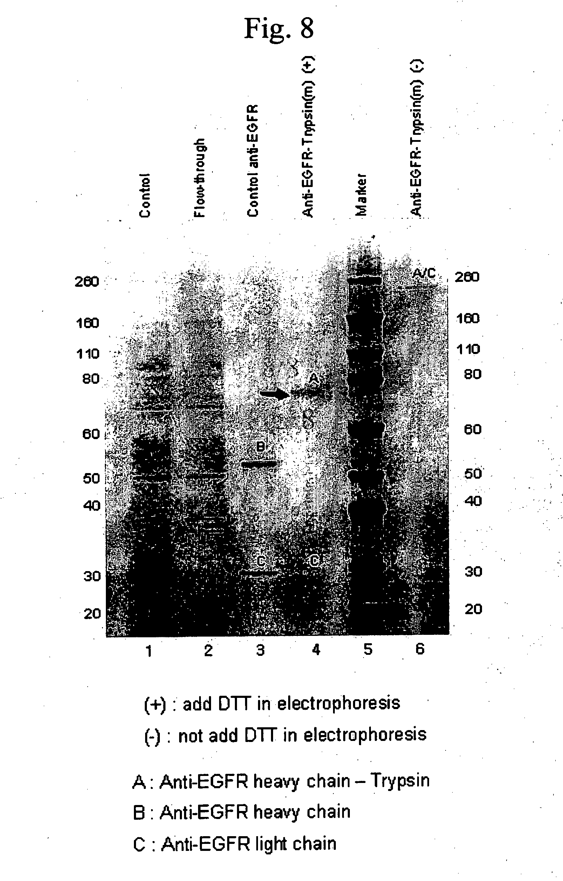

Various solutions from the antibody purification process were analyzed by protein gel electrophoresis. The results are shown in Fig. 8. In Fig. 8, Lane 5 contains size markers. Lane 1 is a control, showing the crude cell culture supernatant before application to the column. Lane 2 shows the column flow-though, i.e., proteins which are not bonded to the ecotin after application to the column. Lane 3 is a control anti-EGFR antibody without a trypsin tag purified with a Protein A affinity column, showing the positions of the light (C) and heavy chain (B) bands. Lanes 4 and 6 show the anti-EGFR antibody with a trypsin tag purified with the ecotin affinity chromatography medium column, with or without DTT treatment in electrophoresis, respectively. In lane 4, the trypsin tagged heavy chain band is labeled A and C is the light chain. In lane 6, in the absence of DTT, the chains are associated and run as a single band (labeled A/C) running at a higher molecular weight.

The terminology used herein is for the purpose of describing particular embodiments only. The terms "a" and "an" do not denote a limitation of quantity, but rather denote the presence of at least one of the referenced item. The term "or" means "and/or". The terms "comprising", "having", "including", and "containing" are to be construed as open-ended terms (i.e., meaning "including, but not limited to").

Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable.

All methods described herein can be performed in a suitable order unless otherwise indicated herein or otherwise clearly contradicted by context. The use of any and all examples, or exemplary language (e.g., "such as"), is intended merely to better illustrate the invention and does not pose a limitation on the scope of the invention unless otherwise claimed. No language in the specification should be construed as indicating any non-claimed element as essential to the practice of the invention as used herein. Unless defined otherwise, technical and scientific terms used herein have the same meaning as is commonly understood by one of skill in the art to which this invention belongs.

Preferred embodiments of this invention are described herein, including the best mode known to the inventors for carrying out the invention. Any combination of the above-described elements in all possible variations thereof is encompassed by the invention unless otherwise indicated herein or otherwise clearly contradicted by context.

## Claims

1. A method of purifying a target protein from a sample, comprising
contacting the sample containing a tagged target protein to a support having immobilized thereon a protease inhibitor,
wherein the protease inhibitor is ecotin, papaya Kunitz-type trypsin inhibitor, pepstatin A, leupeptin, Gly-Gly-Tyr-Arg, a2-macroglobulin, a2-antiplasmin, antithrombin III human, a1-antitrypsin, bdellin, pepsinostreptin, chymostatin, phosphoramidon, isoamylphosphonyl-Gly-L-Pro-L-Ala, or dipotassium 2(R)-2-mercaptomethyl-4-methylpentanoyl-beta-(2-naphthyl)-Ala-Ala amide,
wherein the tagged target protein is an antibody covalently bound to a peptide tag and the peptide tag has specific binding activity for the protease inhibitor,
wherein the peptide tag is
a) derived from a protease, or
b) a fragment of the protease that lacks the proteolysis activity of the protease, a mutant of the protease that lacks the proteolysis activity of the wildtype protease, or a fragment of the mutant, and
wherein the contact is under conditions such that the protease inhibitor binds to the peptide tag;
removing components of the sample that are not bound; and
then removing the target protein from the support.

2. The method of claim 1, wherein removing the target protein from the support comprises
eluting the tagged target protein from the support, wherein eluting the tagged target protein from the support is preferably conducted by elution at a pH that lowers the binding affinity between the peptide tag and the protease inhibitor such that the tagged target protein is removed from the support; followed by separating the peptide tag from the tagged target protein to obtain the target protein, wherein separating the peptide tag from the tagged target protein preferably comprises treating the eluted tagged target protein with a protease, such as TEV protease, papain, or pepsin.

3. The method of any one of claims 1 to 2, wherein removing the target protein from the support comprises
separating the bound peptide tag from the tagged target protein to obtain the target protein, preferably by treating the bound tagged target protein with a protease.

4. The method of any one of claims 1-3, wherein the protease preferably is papain, trypsin, pepsin, chymotrypsin, rennin, cathepsin D, thermolysin, elastase, plasmin, thrombin, urokinase, or collagenase.

5. The method of any one of claims 1 to 4, wherein the tagged target protein comprises a fusion protein, preferably obtained by ligating a nucleic acid encoding the peptide tag to a nucleic acid encoding the target protein to obtain a nucleic acid encoding the fusion protein, inserting the nucleic acid encoding the fusion protein into a vector for expression, and expressing the fusion protein in a host cell.

6. A method of preparing a recombinant cell for expression of a tagged target protein, comprising
ligating a nucleic acid encoding a peptide tag to a nucleic acid encoding a target protein to obtain a nucleic acid encoding a tagged target protein;
ligating the nucleic acid sequence encoding the tagged target protein into an expression vector to obtain a recombinant vector; and
transforming a host cell with the recombinant vector, wherein the peptide tag is a mutant trypsin lacking protease activity, and wherein the target protein is an immunoglobulin heavy chain.

7. The method of claim 6, wherein the sequence of the mutant trypsin:
a) comprises a fragment of SEQ ID NO: 2 retaining specific binding activity to a protease inhibitor
b) comprises amino acids 24-244 of SEQ ID NO: 2, or
c) consists of amino acids 16-244 of SEQ ID NO: 2.

8. The method of any one of claims 6 to 7, wherein the immunoglobulin heavy chain is the heavy chain of an anti-EGFR antibody.

9. A fusion protein comprising:
a peptide tag having specific binding activity to a protease inhibitor and a target protein, wherein the target protein is an immunoglobulin heavy chain, and wherein the peptide tag is:
a) derived from a protease selected from papain, trypsin, pepsin, chymotrypsin, rennin, cathepsin D, thermolysin, elastase, plasmin, thrombin, urokinase, and collagenase,
b) a fragment of the protease that lacks the proteolysis activity of the protease, a mutant of the protease that lacks the proteolysis activity of the wildtype protease, or a fragment of the mutant, or
c) a mutant trypsin.

10. The fusion protein of claim 9, wherein the sequence of the mutant trypsin:
a) comprises a fragment of SEQ ID NO: 2 retaining specific binding activity to a protease inhibitor, or
b) comprises amino acids 24-244 of SEQ ID NO: 2, or
c) or comprises amino acids 16 - 244 of SEQ ID NO: 2.

11. A recombinant cell comprising an isolated polynucleotide encoding the fusion protein of claims 9 of 10.

## Patentansprüche

1. Verfahren zum Aufreinigen eines Zielproteins aus einer Probe, umfassend
das Inkontaktbringen der Probe, die ein getaggtes Zielprotein enthält, mit einem Träger, auf dem ein Proteaseinhibitor immobilisiert ist,
wobei der Proteaseinhibitor Ecotin, ein Papaya-Trypsininhibitor vom Kunitz-Typ, Pepstatin A, Leupeptin, Gly-Gly-Tyr-Arg, a2-Makroglobulin, a2-Antiplasmin, humanes Antithrombin III, a1-Antitrypsin, Bdellin, Pepsinostreptin, Chymostatin, Phosphoramidon, Isoamylphosphonyl-Gly-L-Pro-L-Ala oder Dikalium-2(R)-2-mercaptomethyl-4-methylpentanoyl-beta-(2-naphthyl)-Ala-Ala-amid ist,
wobei das getaggte Zielprotein ein kovalent an ein Peptid-Tag gebundener Antikörper ist und das Peptid-Tag eine spezifische Bindungsaktivität für den Proteaseinhibitor hat,
wobei das Peptid-Tag
a) von einer Protease abgeleitet ist, oder
b) ein Fragment der Protease, dem die Proteolyseaktivität der Protease fehlt, eine Mutante der Protease, der die Proteolyseaktivität der Wildtyp-Protease fehlt, oder ein Fragment der Mutante ist, und
wobei der Kontakt unter solchen Bedingungen erfolgt, dass der Proteaseinhibitor an das Peptid-Tag bindet;
das Entfernen von Komponenten der Probe, welche nicht gebunden sind; und anschließend das Entfernen des Zielproteins von dem Träger.

2. Verfahren nach Anspruch 1, wobei das Entfernen des Zielproteins von dem Träger umfasst
das Eluieren des getaggten Zielproteins von dem Träger, wobei das Eluieren des getaggten Zielproteins von dem Träger vorzugsweise durch Elution bei einem pH durchgeführt wird, welcher die Bindungsaffinität zwischen dem Peptid-Tag und dem Proteaseinhibitor verringert, so dass das getaggte Zielprotein von dem Träger entfernt wird; gefolgt von dem Trennen des Peptid-Tags von dem getaggten Zielprotein, um das Zielprotein zu erhalten, wobei das Trennen des Peptid-Tags von dem getaggten Zielprotein vorzugsweise das Behandeln des eluierten getaggten Zielproteins mit einer Protease, wie etwa TEV-Protease, Papain oder Pepsin, umfasst.

3. Verfahren nach einem der Ansprüche 1 bis 2, wobei das Entfernen des Zielproteins von dem Träger das Trennen des gebundenen Peptid-Tags von dem getaggten Zielprotein zum Erhalten des Zielproteins, vorzugsweise durch Behandeln des gebundenen getaggten Zielproteins mit einer Protease umfasst.

4. Verfahren nach einem der Ansprüche 1-3, wobei die Protease vorzugsweise Papain, Trypsin, Pepsin, Chymotrypsin, Rennin, Cathepsin D, Thermolysin, Elastase, Plasmin, Thrombin, Urokinase oder Collagenase ist.

5. Verfahren nach einem der Ansprüche 1 bis 4, wobei das getaggte Zielprotein ein Fusionsprotein umfasst, das vorzugsweise durch Ligieren einer Nucleinsäure, die das Peptid-Tag codiert, an eine Nucleinsäure, die das Zielprotein codiert, um eine Nucleinsäure zu erhalten, die das Fusionsprotein codiert, Einfügen der Nucleinsäure, die das Fusionsprotein codiert, in einen Vektor zur Expression, und Exprimieren des Fusionsproteins in einer Wirtszelle erhalten wird.

6. Verfahren zum Herstellen einer rekombinanten Zelle zur Expression eines getaggten Zielproteins, umfassend
das Ligieren einer Nucleinsäure, die ein Peptid-Tag codiert, an eine Nucleinsäure, die ein Zielprotein codiert, um eine Nucleinsäure zu erhalten, die ein getaggtes Zielprotein codiert;
das Ligieren der Nucleinsäuresequenz, die das getaggte Zielprotein codiert, in einen Expressionsvektor, um einen rekombinanten Vektor zu erhalten; und
das Transformieren einer Wirtszelle mit dem rekombinanten Vektor, wobei das Peptid-Tag ein mutiertes Trypsin ist, dem die Proteaseaktivität fehlt, und wobei das Zielprotein eine schwere Kette eines Immunglobulins ist.

7. Verfahren nach Anspruch 6, wobei die Sequenz des mutierten Trypsins:
a) ein Fragment von SEQ ID NO: 2 umfasst, das eine spezifische Bindungsaktivität an einen Proteaseinhibitor beibehält
b) die Aminosäuren 24-244 von SEQ ID NO: 2 umfasst, oder
c) aus den Aminosäuren 16-244 von SEQ ID NO: 2 besteht.

8. Verfahren nach einem der Ansprüche 6 bis 7, wobei die schwere Kette des Immunglobulins die schwere Kette eines Anti-EGFR-Antikörpers ist.

9. Fusionsprotein, umfassend:
ein Peptid-Tag mit einer spezifischen Bindungsaktivität an einen Proteaseinhibitor und ein Zielprotein, wobei das Zielprotein eine schwere Kette eines Immunglobulins ist, und wobei das Peptid-Tag:
a) von einer Protease, ausgewählt aus Papain, Trypsin, Pepsin, Chymotrypsin, Rennin, Cathepsin D, Thermolysin, Elastase, Plasmin, Thrombin, Urokinase und Collagenase, abgeleitet ist,
b) ein Fragment der Protease, dem die Proteolyseaktivität der Protease fehlt, eine Mutante der Protease, der die Proteolyseaktivität der Wildtyp-Protease fehlt, oder ein Fragment der Mutante ist, oder
c) ein mutiertes Trypsin ist.

10. Fusionsprotein nach Anspruch 9, wobei die Sequenz des mutierten Trypsins:
a) ein Fragment von SEQ ID NO: 2 umfasst, das eine spezifische Bindungsaktivität an einen Proteaseinhibitor beibehält, oder
b) die Aminosäuren 24-244 von SEQ ID NO: 2 umfasst, oder
c) die Aminosäuren 16-244 von SEQ ID NO: 2 umfasst.

11. Rekombinante Zelle, umfassend ein isoliertes Polynucleotid, das das Fusionsprotein der Ansprüche 9 oder 10 codiert.

## Revendications

1. Procédé de purification d'une protéine cible à partir d'un échantillon, comprenant
la mise en contact de l'échantillon contenant une protéine cible marquée, avec un support sur lequel a été immobilisé un inhibiteur de protéase,
dans lequel l'inhibiteur de protéase est l'écotine, un inhibiteur de protéase de type Kunitz de papaye, la pepstatine A, la leupeptine, Gly-Gly-Tyr-Arg, l'a2-macroglobuline, l'a2-antiplasmine, l'antithrombine III humaine, l'a1-antitrypsine, la bdelline, la pepsinostreptine, la chymostatine, le phosphoramidon, l'isoamylphosphonyl-Gly-L-Pro-L-Ala, ou le 2(R)-2-mercaptométhyl-4-méthylpentanoyl-bêta-(2-naphtyl)-Ala-Ala amide de potassium,
dans lequel la protéine cible marquée est un anticorps lié par covalence à un marqueur peptidique et le marqueur peptidique a une activité de liaison spécifique vis-à-vis de l'inhibiteur de protéase,
dans lequel le marqueur peptidique est
a) obtenu à partir d'une protéase, ou
b) un fragment de la protéase qui est dépourvu de l'activité de protéolyse de la protéase, un mutant de la protéase qui est dépourvu de l'activité de protéolyse de la protéase de type sauvage, ou un fragment du mutant, et
dans lequel le contact se fait dans des conditions telles que l'inhibiteur de protéase se lie au marqueur peptidique ;
l'enlèvement des composants de l'échantillon qui ne sont pas liés ; et ensuite, l'enlèvement de la protéine cible du support.

2. Procédé de la revendication 1, dans lequel l'enlèvement de la protéine cible du support comprend
l'élution de la protéine cible marquée du support, ladite élution de la protéine cible marquée du support étant de préférence effectuée par élution à un pH qui diminue l'affinité de liaison entre le marqueur peptidique et l'inhibiteur de protéase de sorte que la protéine cible marquée s'enlève du support ; puis la séparation du marqueur peptidique d'avec la protéine cible marquée pour obtenir la protéine cible, ladite séparation du marqueur peptidique d'avec la protéine cible marquée comprend de préférence le traitement de la protéine cible marquée éluée avec une protéase, telle que la protéase TEV, la papaïne, ou la pepsine.

3. Procédé de l'une quelconque des revendications 1 à 2, dans lequel l'enlèvement de la protéine cible du support comprend
la séparation du marqueur peptidique lié d'avec la protéine cible marquée pour obtenir la protéine cible, de préférence par traitement de la protéine cible marquée liée avec une protéase.

4. Procédé de l'une quelconque des revendications 1 à 3, dans lequel la protéase est de préférence la papaïne, la trypsine, la pepsine, la chymotrypsine, la rénine, la cathepsine D, la thermolysine, l'élastase, la plasmine, la thrombine, l'urokinase, ou la collagénase.

5. Procédé de l'une quelconque des revendications 1 à 4, dans lequel la protéine cible marquée comprend une protéine de fusion, de préférence obtenue par ligature d'un acide nucléique codant pour le marqueur peptidique à un acide nucléique codant pour la protéine cible pour obtenir un acide nucléique codant pour la protéine de fusion, insertion de l'acide nucléique codant pour la protéine de fusion dans un vecteur pour expression, et expression de la protéine de fusion dans une cellule hôte.

6. Procédé de préparation d'une cellule recombinante pour l'expression d'une protéine cible marquée, comprenant
la ligature d'un acide nucléique codant pour un marqueur peptidique à un acide nucléique codant pour une protéine cible pour obtenir un acide nucléique codant pour une protéine cible marquée ;
la ligature de la séquence d'acide nucléique codant pour la protéine cible marquée dans un vecteur d'expression pour obtenir un vecteur recombinant ; et
la transformation d'une cellule hôte avec le vecteur recombinant, où le marqueur peptidique est une trypsine mutante dépourvue d'activité protéasique, et où la protéine cible est une chaîne lourde d'immunoglobuline.

7. Procédé de la revendication 6, dans lequel la séquence de la trypsine mutante :
a) comprend un fragment de SEQ ID NO: 2 conservant une activité de liaison spécifique vis-à-vis d'un inhibiteur de protéase
b) comprend les acides aminés 24 à 244 de SEQ ID NO: 2, ou
c) est constituée des acides aminés 16 à 244 de SEQ ID NO: 2.

8. Procédé de l'une quelconque des revendications 6 à 7, dans lequel la chaîne lourde d'immunoglobuline est la chaîne lourde d'un anticorps anti-EGFR.

9. Protéine de fusion comprenant :
un marqueur peptidique ayant une activité de liaison spécifique vis-à-vis d'un inhibiteur de protéase et une protéine cible, ladite protéine cible étant une chaîne lourde d'immunoglobuline, et ledit marqueur peptidique étant :
a) obtenu à partir d'une protéase sélectionnée parmi la papaïne, la trypsine, la pepsine, la chymotrypsine, la rénine, la cathepsine D, la thermolysine, l'élastase, la plasmine, la thrombine, l'urokinase, et la collagénase,
b) un fragment de la protéase qui est dépourvu de l'activité de protéolyse de la protéase, un mutant de la protéase qui est dépourvu de l'activité de protéolyse de la protéase de type sauvage, ou un fragment du mutant, ou
c) une trypsine mutante.

10. Protéine de fusion de la revendication 9, dans laquelle la séquence de la trypsine mutante :
a) comprend un fragment de SEQ ID NO: 2 conservant une activité de liaison spécifique vis-à-vis d'un inhibiteur de protéase, ou
b) comprend les acides aminés 24 à 244 de SEQ ID NO: 2, ou
c) ou comprend les acides aminés 16 à 244 de SEQ ID NO: 2.

11. Cellule recombinante comprenant un polynucléotide isolé codant pour la protéine de fusion des revendications 9 ou 10.
